# DEMANDE DE BREVET EUROPEEN

(11) **EP 1 075 819 A1**
(43) Date de publication de la demande: **14.02.2001**
(21) Numéro de dépôt: 00500185.4
(22) Date de dépôt: 10.08.2000
(51) Int. Cl.: A61B 17/32, A45D 29/00, A61B 17/54

(54) **Dispositif de libération de la lame d'un coupe-callosité**

(30) Priorité: 11.08.1999 ES 9900021
(71) Demandeur: Bermejo Martinez, Inmaculada, 01320 Oyon, Alava (ES)
(72) Inventeur: Bermejo Martinez, Inmaculada, 01320 Oyon, Alava (ES)
(74) Mandataire: Manzano Cantos, Gregorio

(57) **Abrégé**

Comprenant quelques éléments mâles de charnière latéraux pour l'articulation de la pièce mobile de serrage de la lame de coupe, pourvu d'extensions aussi latérales coiffées sur une touche, faisant l'office de poussoir, permettant d'ouvrir le support de ladite lame en le faisant basculer sur des moyens respectifs de pivotement latéral, sans aucun risque de coupe et ayant un point d'enclenchement pour maintenir fermer ledit support lorsque la liberation de ladite lame n'est pas nécessaire.

## Description

### MÉMOIRE DESCRIPTIF

L'invention selon l'exposé concerne un dispositif pour libérer et placer automatiquement, et sans aucun risque pour l'utilisateur, la lame de coupe utilisée par l'utensil coupe-callosité pour sa fonction spécifique de coupe.

L'invention a par conséquent comme avantage principal, la facilité, la rapidité et l'absence de risque de coupe lors de l'ouverture et la fermeture des pièces pour changer la lame en incorporant un dispositif au moyen duquel la pièce support de la lame et la pièce serrée sont unies au moyen d'une charnière à glissière dont l'ouverture et la femeture sont réalisées au moyen d'un poussoir à distance de la lame en matériel plastique au toucher doux.

### ANTÉCÉDENTS DE L'INVENTION

Il existe sur le marché, fondamentalement, deux types de coupe-callosités.

L'un de fabrication allemande et marque "PEDI" qui est le plus vendu en Europe et qui est vendu depuis plus de 20 ans.

Un coupe-callosité ayant une pièce support pour la lame et une autre de serrage s'emboîte latéralemente contre la première sur laquelle elle se déplace.

La pièce de serrage enfile la lame et l'entraîne vers l'avant et vers l'arrière sur une petite portion pour sorir le tanchant de la lame de coupe de la pièce support ou pour l'escamoter en gardant ce trachant.

Dans un autre modèle fabriqué en Espagne les deux pièces sont unies par une charnière ajustée au moyen d'une vis.

### DESCRIPTION DE L'INVENTION

Dans le modèle, selon l'invention, l'ouverture et la fermeture du changement de la lame est réalisée avec les mêmes mouvements que le système traditionnel connu, mais à partir d'un poussoir ou actionneur à distance du jeu de pièces pour serrer la lame, ce qui fait que l'opération soit beaucoup plus simple et sûre.

Dans le modèle, selon l'invention, pour ouvrir les deux pièces de serrage, il faut actionner le poussoir vers l'arrière (il ne faut pas pousser vers le bas) et lorsque l'on arrive á un point, la pièce mobile bascule sur la charnière en l'ouvrant et en introduisant ou sortant ainsi la lame.

De même, et selon l'objet de l'invention, lorsqu'il s'agit de l'opération de fermeture, il faut presser le poussoir vers le bas tout en forçant les pivots de la charnière vers le haut, pour que les deux pièces de serrage soient accouplées et à la suite on déplace l'actionneur vers l'avant jusqu'à ce que la languette centrale soit en butée.

Les profondeurs de la pièce sont alors les traditionnelles connues, avec la languette horizontale, pour taverser la lame à travers la rainure propre et guider la pièce mobile sur ladite languette.

Nous aborderons plus largement les caractéristiques essentielles de l'invention à la suite, en faisant référence aux feuilles de dessins accompagnant ce mémoire, où d'une façon un peu schématiquement, et uniquement à mode d'exemple, on représente les détails préférés de l'invention.

Dans les dessins:

La figure 1 est une vue en projection latérale de l'ensemble coupe-callosité, selon le modèle.

La figure 2, est une vue en plan supérieure à 90° de la figure 1.

La figure 3 est une vue en plan inférieure à 180° de la figure 2.

La figure 4 est une vue en projection similaire à la figure 1 du coupe-callosité ouvert.

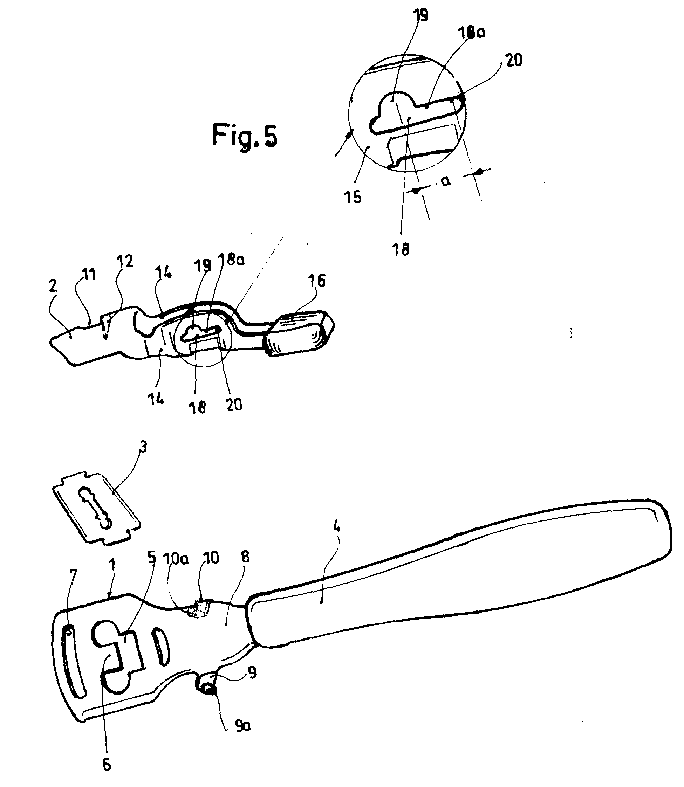

La figure 5 est une vue en perspective démontée coupe-callosité, selon le modèle.

### RÉALISATION PRÉFÉRÉE DE L'INVENTION

Selon une réalisation préférée de l'invention et selon lesdites représentations, l'ensemble de coupe-callosité et ses parties sont identifiées selon les références suivantes:
1. Pièces support fixe,
2. Pièce de serrage mobile
3. Lame
4. Manche de pièce (1)
5. Ouverture de guidage de lame
6. Languette de guidage de pièce mobile (2)
7. Rainure de coupe
8. Extension de support fixe.
9-10. Pattes latérales
9a-10a. Pivots de charnière
11. Rainure de pièce mobile de serrage (2)
12. Oreille de butée de lame (3)
13. Rainure de butée de lame (3)
14-15. Bras actionneur
16. Poussoir actionneur
17-18. Rainure de bras (14-15) de charnière
19. Ouvertures de basculement de charnière
20. Butée d'enclencheent de charnière

Selon ladite représentation la pièce fixe de support (1) et la pièce mobile de serrage (2) sont connues.

L'ouverture de guidage de lame (3) est aussi connue, ainsi que la languette (6) de guidage de la pièce mobile (2) et la rainure (7) de coupe de la pièce (1) .

La rainure (11) est aussi connue, ainsi que l'oreille (12) et la rainure (13) de butée de la pièce mobile de serrage.

L'extension (8) de la pièce de supporte fixe (1) et le jeu de charnières latérales composées par des pattes (9-10) descendantes et particulières des chants de l'extension coiffés sur un pivot en équerre (9a-10a) saillant où bascule la pièce de serrage mobile (2) sont nouveuax et caractéristiques.

Cette pièce (2) a deux bras latéraux parallèles (14-15) qui sont une prolongation des côtés de la pièce (2) et qui sont coiffés sur un poussoir (16) qui est l'organe d'actionnement du guide (2).

Ces bras (14-15) respectivement et centralement ont une rainure allongée (17-18) à silhouette mixte, composée d'une ouverture semi-circulaire à rayon plus grand (19) et prolongation de la rainure à butée (20) à rayon plus petit.

Les rainures (17-18) sont montées sur les pattes (9-10) et guidées sur les appendices de pivotement (9a-10a). Lorsque l'ouverture (19) à rayon plus grand tombe sur les pivots (9a-10a), la pièce de serrage (2) bascule et s'ouvre pour libérer ou placer la lame (3). Lorsque la butée (20) à rayon plus petit tombe sur les pivots (9a-10a) la pièce de serrage (2) reste enclenchée et fermée.

La distance a-a entre les centres de l'ouverture (19) et la butée (20) est égale à la distance entre l'oreille de butée (12) et la languette de guidage (6) lors de son déplacement maximal.

Les rainures (17-18) ont une position intermédiaire où, la lame (3), n'est pas en position de coupe.

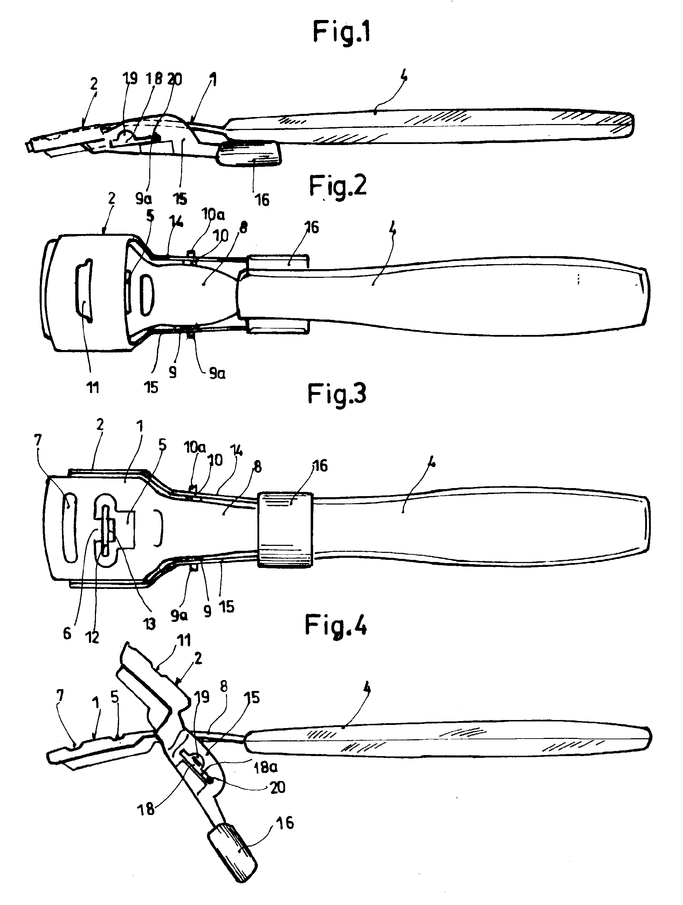

Pour ouvrir la pièce (2) de serrage de la lame (3) il ne faut que tirer du poussoir (16). Lorsque les pivots (9a-10a) tombent sur les ouvertures à rayon plus grand (19) la pièce (2) bascule et elle s'ouvre (figure 4).

Pour fermer la pièce (2) il faut simplement tirer vers le bas du poussoir (16) serrant la lame (3) en posiction intermédaire (17a-18a) des rainures (17-18) et pour sortir la lame (3) en position de coupe, il ne faut que pousser le poussoir (16) jusqu'à ce que les pivots (9a-10a) tombent sur les butées à rayon plus petit (20).

De cette façon on assure, sans aucun risque, le montage-démontage de la lame (3), car il faut simplement la déposer sur la pièce fixe de support (1) pour que sa rainure centrale coïncide avec l'ouverture (5) du supporte (1). Alors on tire du poussoir (16) vers le bas en fermant la pièce de serrage (2) contre la lame (3), en pouvant ainsi la déplacer vers sa position de coupe sur la rainurte (7) en poussant le poussoir (16) vers la butée à rayon plus petit (20).

Une fois décrite convenablement la nature de l'invention, il faut remarquer aux effets opportuns, que celle-ci ne se limite pas aux détails exacts de cet exposé, mais que au contraire, on y introduira les modification considérées opportunees, si toutefois celà ne modifie pas les caractéristiques essentielles de celle-ci, revendiquées à la suite.

## Revendications

1. DISPOSITIF DE LIBÉRATION DE LA LAME D'UN COUPE-CALLOSITÉ, comprenant un organe ou pièce de support fixe (1) uni à un manche respectif (4) et ayant une ouverture centrale (5) pour le guidage de la lame (3) et une languete (6) respective de guidage de la pièce mobile correspondante de serrage (2), superposé, comprenant aussi une rainure centrale (11) ayant une oreille verticale entrante de butée (12) et une rainure de butée correspondante (13) pour positionner la lame de coupe (3) sur la rainure de coupe (7) de la pièce de support (1), CARACTÉRISÉ en ce que ladite pièce de support (1) a une extension (8) entre celle-ci et le manche (4) et des membres mâles (9-10) de charnière latéraux et descendants pour l'articulation de la pièce mobile (2) de serrage de la lame (3), ayant des bras latéraux parallèles (14-15) qui prolongent les côtés de ladite pièce (2), des moyens femelles respectifs (17-18) de charnière étant coiffés sur un poussoir-actionneur (16) à la façon une touche.

2. DISPOSITIF DE LIBÉRATION DE LA LAME D'UN COUPE-CALLOSITÉ, selon la revendication 1, qui comprend des membres (9-10) mâles de charnière CARACTÉRISÉS en ce que des pattes verticales descendantes (9-10) et perpendicualires à l'extension (8) de la pièce de support fixe (1) coifés sur del appendices saillant vers l'extérieur (9a-10a) et en équerre par rapport aux pattes (9-10).

3. DISPOSITIF DE LIBÉRATION DE LA LAME D'UN COUPE-CALLOSITÉ, selon la revendication 1, les bras parallèles (14-15) de la pièce mobile (2) de serrage de la lame (3) sont CARACTÉRISÉS en ce qu'ils comprennet les rainures centrales (17-18) à silhouette mixte, ayant une partie intermédiare (17a-18a) plus fermée, des ouvertures d'extrème à rayon plus grand (19) servant au basculement et à l'ouverture de la pièce (2) et des butées à rayon plus petit (20) pour l'enclenchement de la pièce (2).

4. DISPOSITIF DE LIBÉRATION DE LA LAME D'UN COUPE-CALLOSITÉ, selon la revendication 1, l'élément poussoir (16) est caractérisé en ce qu'il ouvre la pièce de serrage (2) en y exerçant une traction et il met la lame (3) en position de coupe en le posussant légèrement.
